Europäisches Patentamt

European Patent Office

Office européen des brevets

(19)

(11) Publication number: **0 462 066 A1**

# (12) EUROPEAN PATENT APPLICATION

(21) Application number: **91810433.2**

(22) Date of filing: **10.06.91**

(51) Int. Cl.$^5$: **A61K 31/19, A61K 9/10**

(30) Priority: **15.06.90 US 539160**

(43) Date of publication of application:
**18.12.91 Bulletin 91/51**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950 (US)**

(72) Inventor: **Ghebre-Sellassie, Isaac**
**359 Dell Place**
**Stanhope, New Jersey 07874 (US)**
Inventor: **Iyer, Uma**
**7 Florie Farm Road**
**Mendham, New Jersey 07945 (US)**

(74) Representative: **Silbiger, Jakob, Dr.**
**c/o CAPSUGEL AG Fabrikmattenweg 2-4**
**CH-4144 Arlesheim (CH)**

(54) Amorphous gemfibrozil.

(57) A homogeneous solid dispersion of amorphous 5-(2,5-dimethylphenoxy)-2,2-dimethylpentanoic acid and polyvinylpyrrolidone which upon melting does not exhibit transition enthalpy has improved dissolution properties.

EP 0 462 066 A1

The present invention relates to a new physical form of gemfibrozil.

Gemfibrozil, or 5-(2,5-dimethylphenoxy)-2,2-dimethylpentanoic acid, is a widely used antihyperlipoproteinemic agent. Physically the chemical is a crystalline material which melts in the range of 61° to 63°C (hexane) and exhibits a boiling point of $158\text{-}159°_{0.02}C$. Its typical daily dose is high generally, about 1200 mg, probably because of poor water solubility. Because the dose is so high, considerable research has been devoted to formulations which can reduce the size of the dosage form which must be administered. An alternative approach is to increase the bioavailability of the drug, thereby permitting a reduction in the required dosage.

U.S. Patent No. 4,716,033 discloses a medicament adsorbate having a medicament, including inter alia gemfibrozil, and surfactant adsorbed thereon.

U.S. Patent No. 4,753,800 discloses a medicament adsorbate having a medicament, including inter alia gemfibrozil, dispersed in an edible wax adsorbed thereon.

U.S. Patent No. 4,814,354 discloses pharmaceutical compositions of an anion exchange resin lipid regulator, such as cholestyramine or cholestipol, and gemfibrozil.

U.S. Patent No. 4,778,676 discloses a chewable confection delivery system of coated cholestyramine and a confectionery matrix..

U.S. Patent No. 4,816,264 discloses an oral delivery system having a core portion of drug and cellulosic gelling polymer and a semipermeable membrane around the core.

U.S. Patent No. 4,865,850 discloses a method of expelling fat from the gastrointestinal tract by administering non-biodegradable collagen particles having fat receptors, including inter alia gemfibrozil, on their surface.

EP-A 295,637 A2 discloses pharmaceutical compositions in which a lipid regulating component, including inter alia gemfibrozil, is combined with inhibitor of acylCoA:cholesterol acyltransferase.

EP-A 261,693 A1 discloses a lipid regulating component, including inter alia gemfibrozil, which has been pretreated to render it stable until such time as it reaches the proximal section of the intestines.

PCT WO 88/05296 discloses pharmaceutical compositions in which a lipid regulating component, including inter alia gemfibrozil, is combined with inhibitor of 3-hydroxy-3-methylglutaryl coenzyme A reductase.

The present invention involves a new physical form of gemfibrozil having an improved dissolution rate and thus greater bioavailability.

According to the present invention there is provided a homogeneous solid dispersion which does not exhibit transition enthalpy upon melting, comprising amorphous 5-(2,5-dimethylphenoxy)-2,2-dimethylpentanoic acid and polyvinylpyrrolidone, wherein the weight ratio of 5-(2,5-dimethylphenoxy)-2,2-dimethylpentanoic acid to polyvinylpyrrolidone is from about 10:1 to about 1:10.

The present invention also relates to a medicament comprising said dispersion, for the treatment of hyperlipoproteinemia.

The present invention still further relates to the use of said dispersion in the preparation of a medicament for the treatment of hyperlipoproteinemia.

The present invention relates to amorphous 5-(2,5-dimethylphenoxy)-2,2-dimethylpentanoic acid in a homogeneous solid dispersion with polyvinylpyrrolidone. This solid dispersion does not exhibit transition enthalpy upon melting.

Hence rather than demonstrating a discontinuity in enthalpy at phase transition from solid to liquid, as is characteristic of first-order phase transitions for solids possessing a latent heat of fusion, the present solid dispersion exhibits substantially continuity of enthalpy at phase transition. This is apparent from the drawings in which:

FIGURE 1 is a differential scanning calorimeter plot (cal./min v T) for crystalline gemfibrozil, and

FIGURE 2 is a differential scanning calorimeter plot for remelted gemfibrozil; and

FIGURE 3 is an analogous differential scanning calorimeter plot for a typical solid dispersion of gemfibrozil according to the present invention.

The weight ratio of 5-(2,5-dimethylphenoxy)-2,2-dimethylpentanoic acid to polyvinylpyrrolidone will range from about 10:1 to 1:10, particularly from 2:1 to about 1:2. A preferred ratio is about 1:1. The solid dispersion is readily prepared by melting crystalline gemfibrozil, mixing the appropriate amount of polyvinylpyrrolidone, and allowing the mixture to cool. The cooled solid dispersion then can be converted to a particulate by conventional milling techniques and the particulate granulated and formulated, as for example by filling conventional capsules, compressing into tablets, or simply converting it into a dispersible powder form by adding the usual excipients.

Alternately, the solid dispersion can be prepared by dissolving gemfibrozil and PVP in the desired ratio in a conventional organic solvent, and allowing the solvent to evaporate completely.

Solid dispersion is used herein to denote the homogeneous physical nature of the material which should be distinguished from matrix formulations in which active ingredient is located in polymeric matrices, presenting a clearly discernable interface between the two components. As noted, the present material is homogeneous with no interface between the components.

From studies to date, it appears polyvinylpyrrolidone and gemfibrozil are unique in their ability to form solid dispersions which do not exhibit first-order phase transition. While a variety of other materials can be mixed with crystalline gemfibrozil, the resulting mixtures demonstrate a first-order phase transition similar to that seen with the crystalline material.

The solid dispersion is used, either as a solid or particulate, in precisely the same fashion as crystalline gemfibrozil in formulating pharmaceutical compositions. As discussed below, however, the solid dispersion exhibits a significantly improved rate of dissolution, thereby increasing bioavailability.

In formulating pharmaceutical compositions, various excipients can be combined with the solid dispersion. These include fillers such as starch, cellulose derivative, sugars, and similar polysaccharides; separating or anti-tackiness agents such as kaolin, talc, magnesium trisilicate, silicon dioxide, calcium carbonate and the like; plasticizers; stabilizers; lubricants such as magnesium stearate, calcium stearate, zinc stearate, colloidal silicon dioxide, stearic acid, polyethylene glycol, and the like; surfactants such as polysorbates and sodium lauryl sulfate, and the like.

The following example will serve further to typify the nature of the invention but this should not be construed as being a limitation on the scope thereof, which scope is defined by the appended claims.

Example

One hundred parts by weight of 5-(2,5-dimethylphenoxy)-2,2-dimethylpentanoic acid are heated in a steam jacketed mixer until molten. There then is added 100 parts by weight of polyvinylpyrrolidone and the two are intimately mixed. The resulting solid dispersion is allowed to cool.

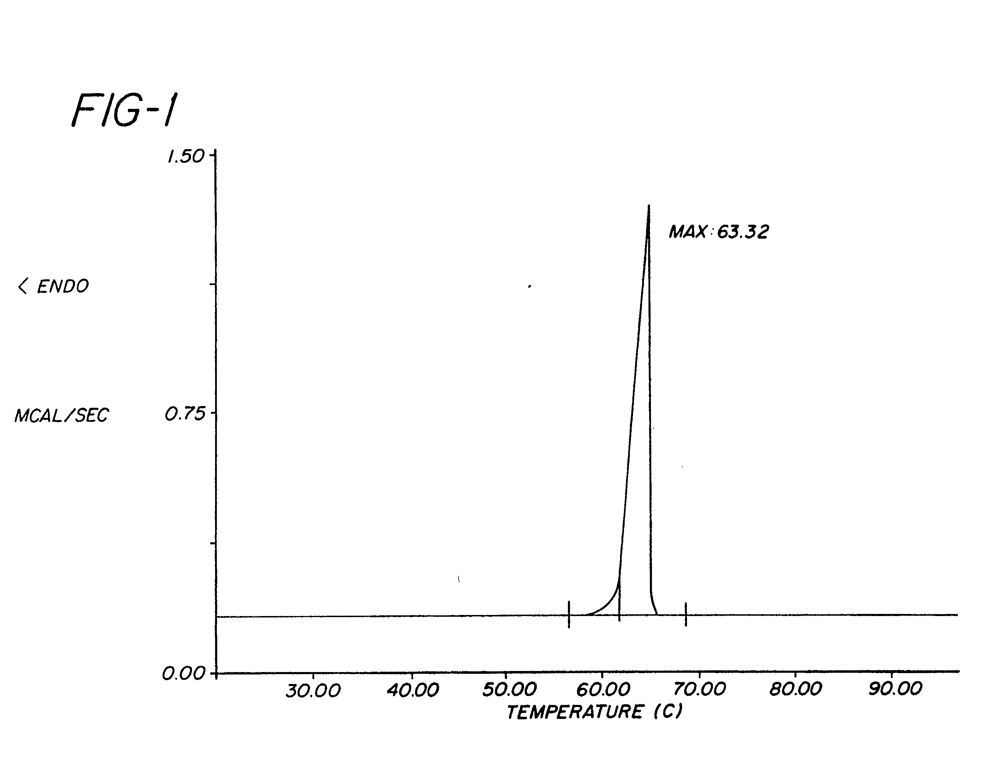

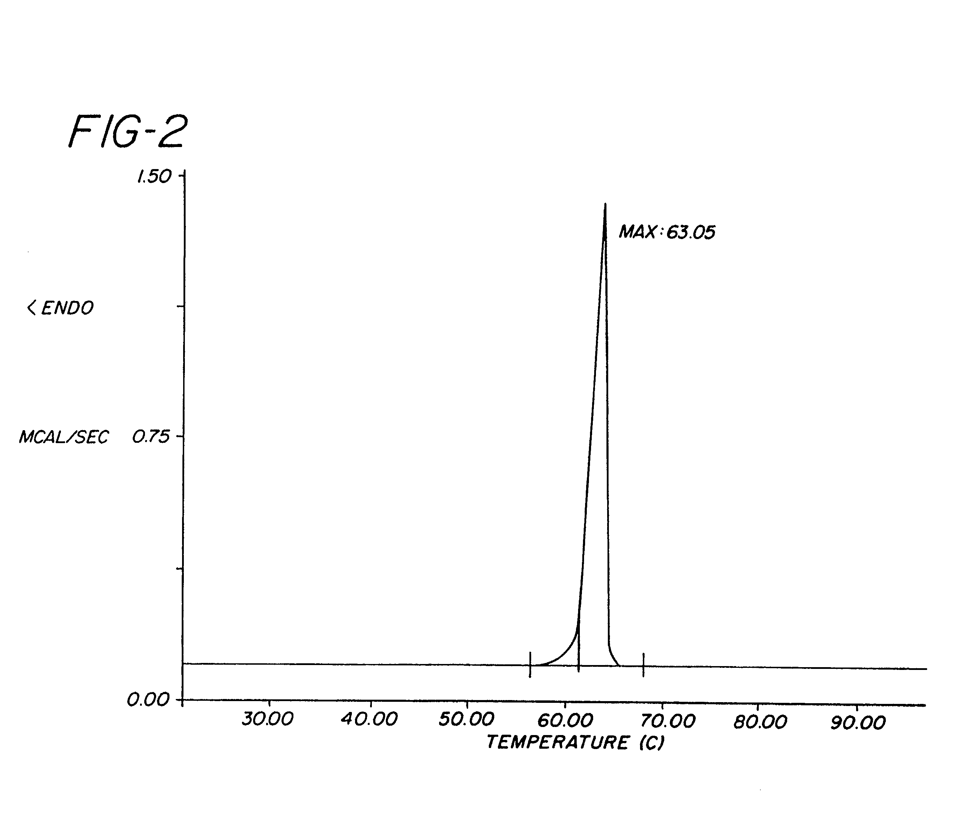

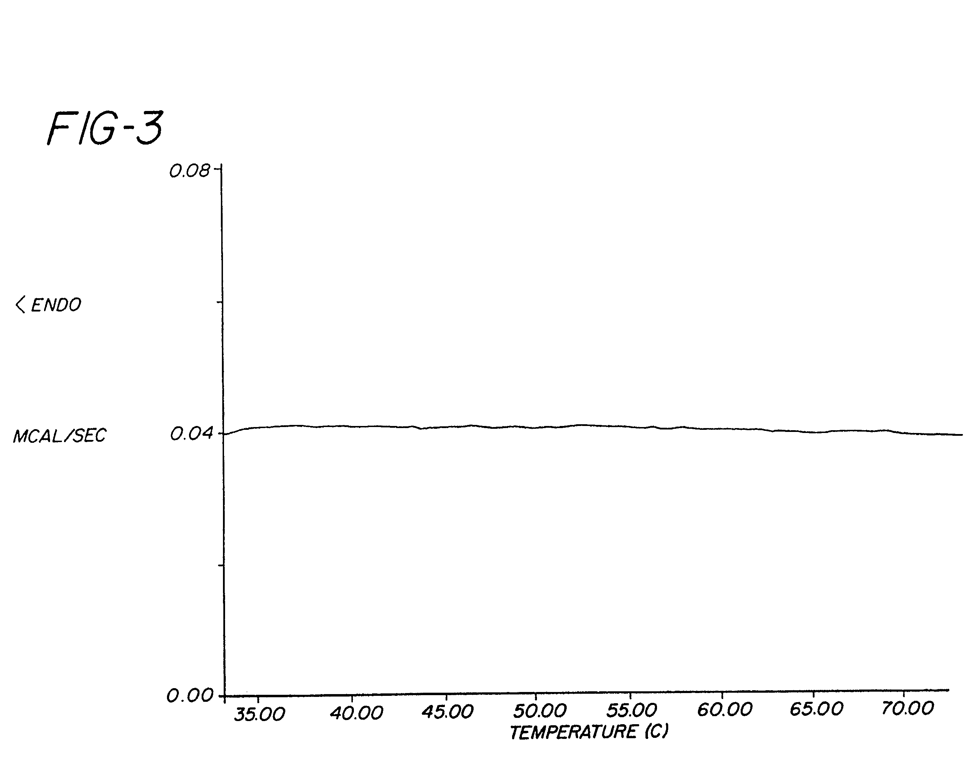

This solid dispersion while turning liquid in the area of 55°-60°C does not exhibit a melting point and does not exhibit transition enthalpy in this phase transition, as shown in Figure 3. In contrast, and as shown in Figure 1 and 2, crystalline material, even that which has been remelted, exhibits a peak on the DSC plot, indicating first-order transition.

In Figure 1 for example, increasing the temperature at a rate of 5.00°/min. produces an endothermic peak (corresponding to the heat of fusion) of 24.83 cal./gram between 56.41° and 68.31°C.

Similarly, and as shown in Figure 2, gemfibrozil which has been melted and cooled to resolidify demonstrates an endothermic peak of 24.41 cal./gram between 56.62° and 68.11°C (5.00°/min.).

No peak is observed, however, for a 1:1 solid dispersion of gemfibrozil and polyvinylpyrrolidone, as shown in Figure 3. (It may be noted that polyvinyl- pyrrolidone itself is thermally inactive over the range tested.)

The foregoing solid dispersion can be milled and used in otherwise conventional gemfibrozil pharmaceutical formulations such as tablets, capsules, and dispersible powder forms.

The superior rate of dissolution of this new form can be seen from the following data in which crystalline 5-(2,5-dimethylphenoxy)-2,2-dimethylpentanoic acid is compared with the solid dispersion.

| Time | Solubility (mcg/ml) | |
| --- | --- | --- |
| | Crystalline | Solid Dispersion |
| One hour | 14.71 | 31.45 |
| Two Hours | 18.43 | 36.85 |
| Twenty Hours | 21.30 | 54.92 |
| Twenty-four Hours | 22.41 | 55.29 |

As can be seen from the foregoing, the dissolution rate in water for the solid dispersion is at least twice that of the crystalline material.

The present invention, thus having been described, is summarised by the following clauses, numbered 1-6, and is defined by the claims appended thereafter.

1. Amorphous 5-(2,5-dimethylphenoxy)-2,2-dimethylpentanoic acid in a homogeneous solid dispersion with polyvinylpyrrolidone wherein the weight ratio of 5-(2,5-dimethylphenoxy)-2, 2-dimethylpentanoic acid to polyvinylpyrrolidone of from about 10:1 to about 1:10, which dispersion upon melting does not exhibit transition enthalpy.

2. Amorphous 5-(2,5-dimethylphenoxy)-2,2-dimethylpentanoic acid according to 1, wherein the weight ratio of 5-(2,5-dimethylphenoxy)-2,2-dimethylpentanoic acid to polyvinylpyrrolidone is from about 2:1 to about 1:2.

3. Amorphous 5-(2,5-dimethylphenoxy)-2,2-dimethylpentanoic acid according to 1, wherein the weight ratio of 5-(2,5-dimethylphenoxy)-2,2-dimethylpentanoic acid to polyvinylpyrrolidone is about 1:1.

4. Amorphous 5-(2,5-dimethylphenoxy)-2,2-dimethylpentanoic acid according to 1, wherein the homogeneous solid dispersion is in particulate form.

5. In an oral pharmaceutical composition containing 5-(2,5-dimethylphenoxy)-2,2-dimethylpentanoic acid as an active antihyperlipoproteinemic component, the improvement which consists of employing said 5-(2,5-dimethylphenoxy)-2,2-dimethylpentanoic acid in the physical form of the homogeneous solid dispersion according to 1.

6. The improvement according to 5, wherein the pharmaceutical composition is in tablet, capsule, or dispersible powder form.

## Claims

1. A homogeneous solid dispersion which does not exhibit transition enthalpy upon melting, comprising amorphous 5-(2,5-dimethylphenoxy)-2,2-dimethylpentanoic acid and polyvinylpyrrolidone, wherein the weight ratio of 5-(2,5-dimethylphenoxy)-2,2-dimethylpentanoic acid to polyvinylpyrrolidone is from about 10:1 to about 1:10.

2. A dispersion according to claim 1, wherein the weight ratio of 5-(2,5-dimethylphenoxy)-2,2-dimethylpentanoic acid to polyvinylpyrrolidone is from about 2:1 to about 1:2.

3. A dispersion according to claim 1, wherein the weight ratio of 5-(2,5-dimethylphenoxy)-2,2-dimethylpentanoic acid to polyvinylpyrrolidone is about 1:1.

4. A dispersion according to any preceding claim, when in particulate form.

5. A medicament comprising the dispersion of any preceding claim.

6. A medicament according to the preceding claim, when used in the treatment of hyperlipoproteinemia.

7. A medicament according to either of the preceding claims, in the form of an oral composition, tablet, capsule, or dispersible powder

8. The use of the dispersion of any one of claims 1 - 4, in the preparation of a medicament for the treatment of hyperlipoproteinemia.

9. The use according to claim 8, in which the dispersion is in the form of an oral pharmaceutical composition.

10. The use according to claim 8, wherein the dispersion is in tablet, capsule, or dispersible powder form.

4

EP 0 462 066 A1

# FIG-1

MAX: 63.32

< ENDO

MCAL/SEC

Y-axis: 0.00, 0.75, 1.50

X-axis (TEMPERATURE (C)): 30.00, 40.00, 50.00, 60.00, 70.00, 80.00, 90.00

FIG-2

EP 0 462 066 A1

FIG-3

EP 0 462 066 A1

European Patent
Office

**EUROPEAN SEARCH REPORT**

Application Number

EP    91 81 0433

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-248447 (WARNER-LAMBERT)<br>* page 3, lines 16 - 18 * | 1-10 | A61K31/19<br>A61K9/10 |
| D | & US-A-4816264 | | |
| A | GB-A-2157170 (CIBA-GEIGY)<br>* abstract * | 1-10 | |
| A,P | EP-A-381219 (WARNER-LAMBERT)<br>* page 2, line 46 *<br>* page 3, line 21 * | 1-10 | |
| A,P | EP-A-381218 (WARNER-LAMBERT)<br>* page 2, line 50 *<br>* page 3, line 20 * | 1-10 | |
| | | | **TECHNICAL FIELDS SEARCHED (Int. Cl.5)**<br><br>A61K |

The present search report has been drawn up for all claims

| Place of search | Date of completion of the search | Examiner |
|---|---|---|
| BERLIN | 01 OCTOBER 1991 | AVEDIKIAN P. F. |

EPO FORM 1503 03.82 (P0401)